(19) Europäisches Patentamt
European Patent Office
Office européen des brevets

(11) EP 3 730 048 A1

(12) DEMANDE DE BREVET EUROPEEN

(43) Date de publication:
**28.10.2020 Bulletin 2020/44**

(21) Numéro de dépôt: **20171261.9**

(22) Date de dépôt: **24.04.2020**

(51) Int Cl.:
*A61B 5/04* (2006.01) *A61B 5/0402* (2006.01)
*A61B 5/048* (2006.01) *A61B 5/16* (2006.01)
*A61B 5/00* (2006.01)

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**
Etats d'extension désignés:
**BA ME**
Etats de validation désignés:
**KH MA MD TN**

(30) Priorité: **26.04.2019 FR 1904422**

(71) Demandeurs:
- **THALES**
**92400 Courbevoie (FR)**
- **UNIVERSITE DE BORDEAUX**
**33000 Bordeaux (FR)**
- **Institut Polytechnique de Bordeaux**
**33402 Talence Cedex (FR)**
- **Centre National de la Recherche Scientifique**
**75016 Paris (FR)**

(72) Inventeurs:
- **LEGRAND, Pierrick**
**33405 Talence (FR)**
- **GRIVEL, Eric**
**33400 Talence (FR)**
- **ANDRE, Jean-Marc**
**33400 Talence (FR)**
- **BERTHELOT, Bastien**
**33700 Mérignac (FR)**

(74) Mandataire: **Marks & Clerk France**
**Immeuble "Visium"**
**22, avenue Aristide Briand**
**94117 Arcueil Cedex (FR)**

# (54) DISPOSITIF ET PROCEDE D'ANALYSE DE L'ETAT D'UN SYSTEME EN CONTEXTE BRUITE

(57) La présente invention concerne un procédé mis en œuvre par ordinateur pour déterminer l'état d'un système, qui comprend des étapes consistant à :
- collecter des données relatives à un système, lesdites données étant des données bruitées comprenant des données d'intérêt et du bruit ;
- générer un signal à analyser à partir des données collectées, ledit signal étant un signal bruité comprenant un signal d'intérêt et du bruit ;
- effectuer une analyse de régularité du signal d'intérêt en compensant l'influence du bruit dans le calcul de la puissance de la différence entre le signal bruité intégré et sa tendance ; et
- déterminer l'état dudit système en fonction du résultat de l'analyse de régularité du signal d'intérêt.

101
Système
100
102
Acquisition données bruitées
104
Génération d'un signal à analyser
106
Estimation de la régularité du signal d'intérêt avec compensation du bruit
108
Détermination de l'état du système
110
Interface utilisateur I/O

Figure 1

EP 3 730 048 A1

## Description

### Domaine de l'invention

L'invention est dans le domaine de l'analyse de l'état d'un système, et en particulier, l'invention concerne un dispositif et un procédé permettant de compenser l'influence d'un bruit additif dans l'analyse de régularité d'un signal.

### Etat de la Technique

Dans un environnement donné, il peut être nécessaire de disposer d'informations sur l'état psycho-physiologique d'une personne ou l'état d'endommagement d'un équipement (structure métallique, pièce de moteur, pont, etc.). Pour ce faire, un ensemble de capteurs recueille des informations de la personne ou de l'équipement et délivre des signaux qui après analyse permettent d'attester et classifier l'état de la personne ou de l'équipement.

A partir des signaux issus des capteurs, des signatures sont extraites. Ces dernières caractérisent les propriétés statistiques, temporelles ou fréquentielles des séries temporelles étudiées.

Cependant, les signaux issus des capteurs sont entachés d'un bruit de mesure qui peut modifier l'information d'intérêt et la valeur des signatures extraites, et de fait entraîner des conséquences néfastes sur leur exploitation.

Une signature particulière porte sur l'analyse temporelle du signal, dont notamment sa régularité notée '$\alpha$'. Cette dernière retranscrit une invariance de comportement du signal par changement d'échelle temporelle. Pour connaître la régularité d'un signal, Il est commun d'estimer le coefficient ou index de Hurst, noté 'H'.

Or, les méthodes connues d'estimation du coefficient de Hurst, comme la méthode dite « Detrended Fluctuation Analysis » (DFA) ou la méthode dite « Detrending Moving Average » (DMA) fournissent une estimation erronée de la signature lorsque les signaux sont perturbés par un bruit. Un bruit peut par exemple être un bruit blanc ou un bruit additif.

Pour pallier ce problème, une approche peut être de rehausser le signal dans le but de supprimer le bruit, puis d'estimer la régularité sur le signal rehaussé. Cependant, les techniques de débruitage classiquement employées, par exemple en traitement de la parole qui reposent sur des approches de type Kalman, de soustraction spectrale ou qui se fondent sur les ondelettes ne donnent pas de résultats satisfaisants. En effet, l'étape de débruitage augmente la complexité calculatoire et le temps de calcul, ce qui est dommageable dans des applications de monitoring où les aspects temps réel sont essentiels. De plus, cette étape de rehaussement (ou débruitage) altère l'estimation de la régularité du signal d'intérêt, en ce que la valeur estimée de $\alpha$ sur le signal rehaussé s'en trouve donc faussée. Les techniques de rehaussement connues ne permettent pas de conserver une estimation correcte de la régularité du signal d'intérêt.

Aussi, il est nécessaire de mettre en place des méthodes d'estimation de signatures qui soient robustes au bruit additif. La présente invention répond à ce besoin.

### Résumé de l'invention

Un objet de la présente invention est un procédé d'estimation de la régularité d'un signal d'intérêt en contexte bruité.

Un autre objet de la présente invention est un dispositif qui comprend des moyens permettant de mettre en œuvre un procédé d'estimation de la régularité d'un signal d'intérêt en contexte bruité.

L'invention trouvera des applications avantageuses dans tous les domaines dont par exemple le milieu (bio)médical où l'analyse de l'état psycho-physiologique d'une personne est requise, mais aussi dans tous les domaines industriels nécessitant le contrôle de pièces, que ce soit en aéronautique, automobile, ferroviaire, ou toute autre industrie lourde.

Pour obtenir les résultats recherchés, il est revendiqué des procédés, dispositifs et produit programme d'ordinateur selon différents modes de réalisation.

En particulier, il est proposé un procédé mis en œuvre par ordinateur pour déterminer l'état d'un système. Le procédé comprend des étapes consistant à :

- collecter des données relatives à un système, lesdites données étant des données bruitées comprenant des données utiles et du bruit ;
- générer un signal à analyser à partir des données collectées, ledit signal étant un signal bruité comprenant un signal d'intérêt et du bruit ;
- effectuer une analyse de régularité du signal d'intérêt à partir du signal bruité en compensant l'influence du bruit dans le calcul de la puissance de la différence entre le signal bruité intégré et sa tendance ; et
- déterminer l'état dudit système en fonction du résultat de l'analyse de régularité du signal d'intérêt.

Selon des modes de réalisation du procédé, alternativement ou en combinaison :

- l'étape d'analyse de régularité du signal bruité comprend les étapes de :
    - définir un jeu de valeurs de 'N' :
        - pour chaque valeur de 'N' :
            - calculer la valeur de la puissance de la différence entre le signal bruité intégré et sa tendance relative à la valeur 'N' à partir de la fonction d'autocorrélation du signal bruité ;
            - calculer la valeur de la puissance de la différence entre le signal d'intérêt intégré et sa tendance à partir de la fonction d'autocorrélation du signal d'intérêt ; et
            - estimer la régularité du signal d'intérêt.
- le procédé comprend après l'étape de générer un signal à analyser, une étape de calculer la fonction d'autocorrélation du signal bruité.
- l'étape de calculer et de compenser la valeur de la puissance de la différence entre le signal d'intérêt intégré et sa tendance comprend les étapes de :
    - estimer les propriétés du bruit ;
    - calculer une fonction d'autocorrélation du bruit ;
    - calculer une fonction d'autocorrélation du signal d'intérêt à partir de la fonction d'autocorrélation du bruit et de la fonction d'autocorrélation du signal bruité ; et
    - compenser mathématiquement par les fonctions d'autocorrélation l'influence du bruit dans le calcul de la valeur de la de la différence entre le signal d'intérêt intégré et sa tendance.
- l'étape d'estimation des propriétés du bruit consiste à étudier la stationnarité et/ou la nature colorée ou blanche du bruit et/ou le représenter par un modèle paramétrique.
- l'étape de définir un jeu de valeurs de 'N' consiste à découper le signal bruité en segments de taille 'N' et l'étape d'estimer la régularité du signal d'intérêt peut consister à inclure l'estimation de la tendance du signal bruité intégré pour le jeu de valeurs de 'N'.
- l'étape de définir un jeu de valeurs de 'N' consiste à appliquer un filtre passe-bas d'ordre 'N' au signal bruité intégré et l'étape d'estimer la régularité du signal d'intérêt peut consister à inclure l'estimation de la tendance du signal bruité intégré.
- l'étape de collecter des données consiste à collecter des données depuis des capteurs couplés au système.
- les capteurs sont configurés pour relever des données relatives à la réponse électrique du cerveau (EEG) « ElectroEncephaloGramme » et/ou à la réponse électrique du cœur (ECG) « ElectroCardioGramme » et/ou à la réponse électrodermale et/ou à la pupillométrie.
- les capteurs comprennent au moins un capteur inertiel qui comprend des accéléromètres et des gyromètres.
- le procédé comprend de plus une étape consistant à afficher l'état dudit système en fonction du résultat de l'analyse de régularité.

L'invention couvre aussi un produit programme d'ordinateur, ledit programme d'ordinateur comprenant des instructions de code permettant d'effectuer les étapes du procédé revendiqué, lorsque le programme est exécuté sur un ordinateur.

L'invention couvre de plus un dispositif pour déterminer l'état d'un système, le dispositif comprenant :

- des moyens pour collecter des données relatives à un système, lesdites données étant des données bruitées comprenant des données utiles et du bruit ;
- des moyens pour générer un signal à analyser à partir des données collectées, ledit signal étant un signal bruité comprenant un signal d'intérêt et du bruit ;
- des moyens pour effectuer une analyse de régularité du signal d'intérêt à partir du signal bruité en compensant l'influence du bruit dans le calcul de la puissance de la différence entre le signal bruité intégré et sa tendance ; et

- des moyens pour déterminer l'état dudit système en fonction du résultat de l'analyse de régularité.

Selon un mode de réalisation, le dispositif comprend de plus une interface utilisateur configurée pour fournir l'état du système.

**Description des figures**

Différents aspects et avantages de l'invention vont apparaitre en appui de la description d'un mode préféré d'implémentation de l'invention mais non limitatif, avec référence aux figures ci-dessous, où :

La figure 1 est un schéma bloc illustrant une implémentation du dispositif de l'invention selon un mode de réalisation ;

La figure 2 illustre un enchaînement d'étapes du procédé de l'invention selon un mode de réalisation ;

La figure 3 illustre un enchaînement d'étapes pour estimer la régularité d'un signal d'intérêt, selon un mode de réalisation de l'invention ;

La figure 4 illustre un enchaînement d'étapes pour compenser l'influence du bruit selon un mode de réalisation de l'invention ;

La figure 5 est une représentation de la pente de la régression de points obtenus à partir de différentes tendances d'un même signal, dans le cas où ledit signal n'est pas bruité ;

La figure 6 illustre sur un graphe log(F(N)) en fonction de log(N), l'évolution de différentes valeurs de $\hat{F}^2(N)$ pour différentes valeurs de N par la méthode DFA et par la méthode de l'invention dans un cas d'un signal non bruité et de ce même signal bruité; et

La figure 7 illustre des comparaisons de résultats obtenus par une approche de rehaussement connue et par la méthode de l'invention dans l'estimation de la régularité de signaux synthétiques de régularité connue et maîtrisée, qui ont été bruités.

**Description détaillée de l'invention**

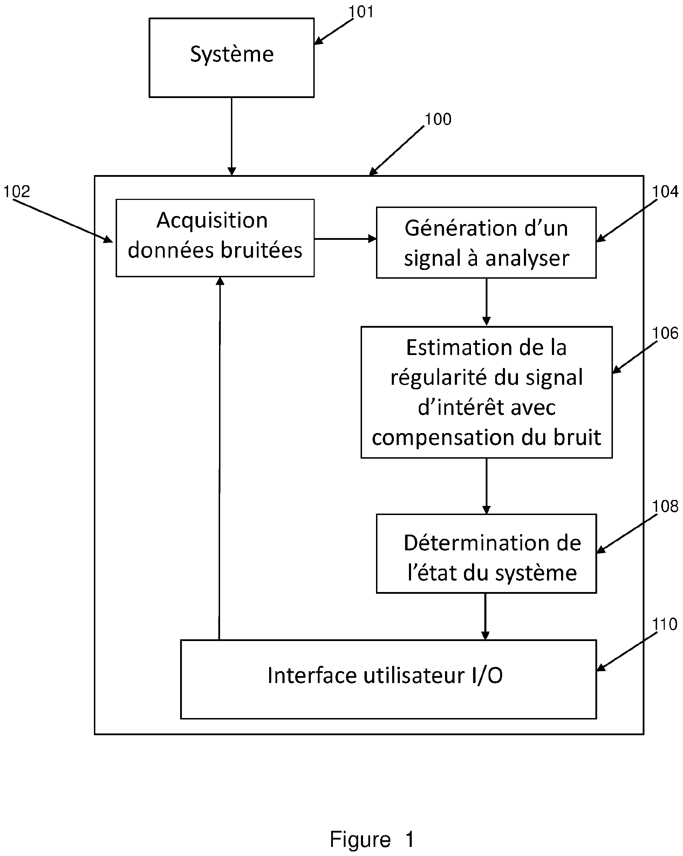

La figure 1 montre une implémentation du dispositif (100) de l'invention, selon un mode de réalisation. Le dispositif (100) comprend généralement un module d'acquisition de données (102) configuré pour recevoir des données d'au moins un capteur apte à enregistrer des informations sur un système surveillé (101), un module de génération de signal (104) configuré pour générer un signal à analyser à partir des données acquises, un module d'analyse (106) configuré selon les principes de l'invention pour estimer la régularité d'un signal d'intérêt à partir du signal bruité et un module de résultat (108) couplé au module d'analyse configuré pour déterminer l'état d'un système à partir des résultats de l'analyse. Le système comprend de plus une interface utilisateur (110) configurée pour fournir le résultat de l'analyse. L'interface utilisateur peut avantageusement être configurée pour paramétrer au besoin le module d'acquisition des données.

La désignation d'un système surveillé dans le cadre de la présente invention doit se comprendre comme pouvant être une personne ou un équipement à surveiller.

La désignation d'au moins un capteur dans le cadre de la présente invention n'est pas limitative en termes de nombre et de nature de capteurs. Ainsi, un ou une pluralité de capteurs peuvent être mis en œuvre pour enregistrer des informations sur le système surveillé.

Dans le cas où le système surveillé est une personne, dans le but de connaître par exemple son niveau d'attention, de somnolence, les capteurs peuvent être des électrodes installées sur la personne afin d'enregistrer des informations relatives à la réponse électrique du cerveau (EEG) « ElectroEncephaloGramme » et/ou à la réponse électrique du cœur (ECG) « ElectroCardioGramme » et/ou à la réponse électrodermale et/ou à la pupillométrie.

Dans le cas où le système surveillé est un équipement, dans le but de connaître par exemple son niveau d'endommagement, les capteurs peuvent permettre d'étudier les forces qui lui sont appliquées et des vibrations qu'il subit. Dans un mode de réalisation, le capteur peut être un capteur inertiel de type « Inertial Measurement Unit » (IMU) qui comprend des accéléromètres et des gyromètres, qui permettent aussi de suivre l'évolution de la position de l'objet dans l'espace.

Le module d'acquisition de données (102) est configuré pour recevoir et enregistrer des données issues des capteurs. La transmission des données depuis les capteurs peut être soit filaire, soit être sans fil. Comme expliqué dans la section de l'état de l'art, les signaux issus des capteurs sont généralement entachés d'un bruit de mesure, bruit lié au contexte de mesure (environnement, capteurs). Les données acquises sont alors des données bruitées comprenant des données utiles et du bruit.

Le module de génération de signal (104) est configuré pour générer une série temporelle à partir des données acquises. Selon des modes de réalisation, les données peuvent être formatées, filtrées, combinées afin de générer un signal à analyser. Les données acquises étant entachées de bruit de mesure additif, le signal à analyser qui est généré, est un signal bruité, composé du signal d'intérêt et du bruit additionnel.

Le module d'analyse (106) est configuré pour estimer selon les principes de l'invention, la régularité du signal d'intérêt. Avantageusement, le procédé de l'invention permet de compenser l'influence du bruit sur l'estimation de la régularité du signal bruité disponible.

Le module de détermination de l'état du système (108) est couplé au module d'estimation de régularité pour déterminer selon le résultat fourni, l'état du système surveillé.

Le dispositif de l'invention comprend une interface utilisateur (110). L'interface utilisateur peut être configurée pour paramétrer le module d'acquisition de données (102). L'interface utilisateur peut être graphique pour visualiser les résultats obtenus par le module de détermination de l'état du système (108). L'interface utilisateur peut comprendre des moyens additionnels pour générer par exemple les résultats de manière audio.

Le dispositif de la présente invention peut s'implémenter à partir d'éléments matériels et/ou logiciels. Le procédé opéré peut être disponible en tant que produit programme d'ordinateur sur un support lisible par ordinateur. Le support peut être électronique, magnétique, optique ou électromagnétique. Dans le cas où l'invention est implantée sur une machine de calcul reprogrammable (par exemple un circuit FPGA), le programme correspondant (c'est-à-dire la séquence d'instructions) peut être stocké dans ou sur un médium de stockage amovible (par exemple une carte SD, ou un stockage de masse tel que un disque dur ex. un SSD) ou non-amovible, volatile ou non-volatile, ce médium de stockage étant lisible partiellement ou totalement par un ordinateur ou un processeur. Le support lisible par ordinateur peut être transportable ou communicable ou mobile ou transmissible (i.e. par un réseau de télécommunication 2G, 3G, 4G, Wifi, BLE, fibre optique ou autre).

Matériellement, le calculateur permettant d'opérer le procédé décrit, peut être implémenté sur tablette ou ordinateur portable. A titre d'exemple d'architecture matérielle adaptée à mettre en œuvre l'invention, un dispositif peut comporter un bus de communication auquel sont reliées une unité centrale de traitement ou microprocesseur (CPU, acronyme de « Central Processing Unit » en anglais), lequel processeur peut être "multi-core" ou "many-core"; une mémoire morte (ROM, acronyme de « Read Only Memory » en anglais) pouvant comporter les programmes nécessaires à la mise en œuvre de l'invention; une mémoire vive ou mémoire cache (RAM, acronyme de « Random Access Memory » en anglais) comportant des registres adaptés à enregistrer des variables et paramètres créés et modifiés au cours de l'exécution des programmes précités ; et une interface de communication ou E/S (I/O acronyme de « Input/ouput » en anglais) adaptée à transmettre et à recevoir des données.

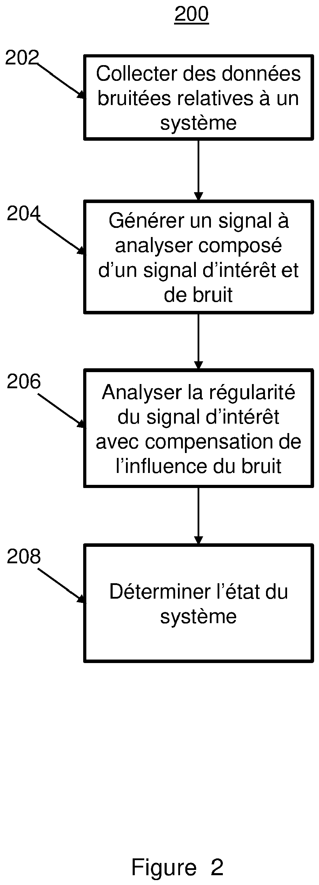

La figure 2 illustre un enchaînement d'étapes (200) pour déterminer l'état d'un système selon les principes de l'invention. Dans une première étape (202), le procédé permet de collecter une pluralité de données du ou des capteurs utilisés pour surveiller un système. Selon un mode de réalisation de l'invention, les données sont enregistrées par le module d'acquisition de données (102) du dispositif (100) de la figure 1. Les données acquises comprennent des données utiles et du bruit.

Dans une étape suivante (204), le procédé permet de générer à partir de tout ou partie des données acquises, un signal. Les données collectées étant bruitées, le signal qui est généré est un signal bruité composé du signal d'intérêt et du bruit. L'objectif est d'estimer la régularité du signal d'intérêt à partir du signal bruité.

L'homme de l'art connait différentes méthodes pour l'analyse de régularité d'un signal, qui se ramènent à deux grandes familles :

- celle des estimateurs reposant sur l'analyse fréquentielle du signal où l'on trouve par exemple la méthode de Whittle locale, la méthode du périodogramme, la méthode fondée sur la transformée en ondelettes ou encore la méthode semi-paramétrique. Plus récemment, des solutions reposant sur « l'Empirical Mode Décomposition » (EMD) ou la transformée de Fourier fractionnelle sont apparues.
- celle des estimateurs reposant sur l'analyse temporelle du signal où l'on trouve par exemple l'analyse appelée « Rescaled Range », la méthode « Aggregated variance », la méthode de « l'absolute-value » ou encore la méthode de la variance du résidu.

Le procédé de l'invention rentre dans la famille de l'analyse temporelle d'un signal, par l'estimation du coefficient de Hurst 'H'. Avant de détailler la méthode revendiquée, il est fait un rappel des approches connues afin d'estimer le coefficient de Hurst d'un processus mono-fractal.

Une méthode nommée en anglais « Fluctuation Analysis » (FA) a été proposée au début des années 1990.

Le principe est le suivant : après intégration d'un signal à analyser, conduisant à une nouvelle séquence '$y_{int}$', il est calculé pour différentes valeurs de 'l' la grandeur suivante : $F_{FA}(l) = \sqrt{< \left(y_{int}(i+l) - y_{int}(i)\right)^2 >}$, où <.> représente la moyenne temporelle.

Puisque $F_{FA}(l) \propto l^{(H+1)}$ avec $\propto$ représentant une relation de proportionnalité, log($F_{FA}(l)$ peut être représenté en fonction de log($l$) afin d'estimer la valeur de 'H' qui est alors égale à la valeur de la pente de la droite de régression obtenue à laquelle on retranche 1.

Plus récemment, deux méthodes dérivées de la méthode (FA) sont largement utilisées pour l'estimation du coefficient de Hurst : la méthode dite « Detrended Fluctuation Analysis » (DFA) et la méthode dite « Detrending Moving Average » (DMA).

La méthode DFA est décrite par C. K. Peng, S. V. Buldyrev, S. Havlin, M. Simons, H. E. Stanley et A. L. Goldberger dans «Mosaic organization of DNA nucleotides» Physical Review, n° %149, pp. 1685-1689, 1994.

La méthode DMA est décrite par D. Osborne dans «Moving average detrending and the analysis of business cycles» Oxford Bull. Econom. Statist, n° %157, pp. 547-558, 1995.

Ces deux approches reposent sur un même principe qui est l'estimation de la tendance du signal intégré, relativement à une grandeur '$N$', en vue d'obtenir la puissance de la différence entre le signal intégré et la tendance. Si le principe est le même pour les deux méthodes, la manière d'estimer la tendance diffère selon l'approche, tout comme la définition de '$N$'.

Pour la méthode DFA, l'estimation de la tendance du signal intégré se fonde sur un découpage du signal en segments de taille '$N$' dont on estime la tendance locale. La tendance globale est alors la concaténation des tendances locales. Pour la méthode DMA, le paramètre '$N$' correspond à l'ordre du filtre passe-bas qui est appliqué au signal intégré pour en obtenir la tendance.

Les étapes de l'analyse de régularité d'un signal selon les approches DFA et DMA sont rappelées ci-après.

En considérant '$M$' échantillons successifs $\{y(m)\}_{m=1,...,M}$ d'un signal '$y$', chaque méthode comprend quatre étapes principales :

Étape 1 : Le signal est intégré et sa moyenne lui est retranchée. Cette étape aboutit à un nouveau signal appelé 'le profil de y et noté :

$$y_{int}(m) = \sum_{i=1}^{m}\left(y(i) - \mu_y\right)$$

où $\mu_y$ désigne la moyenne de $y$.

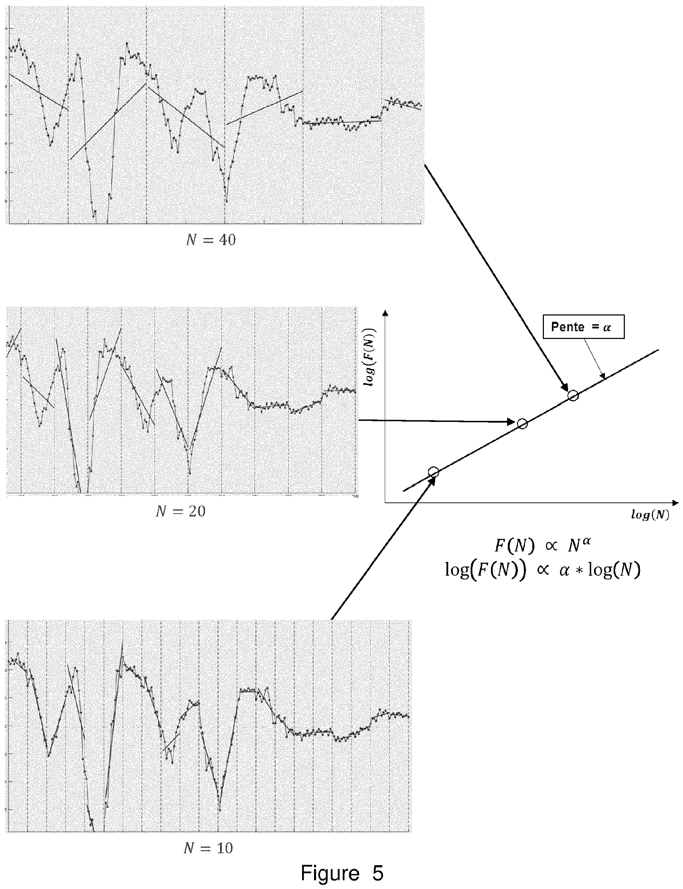

Étape 2 : La tendance du profil est estimée.
Dans le cas de la méthode DFA, le profil est découpé en '$L$' segments de taille '$N$'. Puisque '$M$' le nombre d'échantillons successifs n'est pas nécessairement un multiple de '$N$', on ne prend en compte que $L \times N$ échantillons. La $l$-ième tendance locale ($l$ = 1,...,$L$) est estimée sur le segment $l$ au sens des moindres carrés. La tendance globale du signal intégré est alors définie comme la concaténation des $L$ tendances locales. Un exemple de tendances de profil est illustré sur la partie gauche de la figure 5 pour différentes valeurs de $N$ = (10, 20, 40). Dans le cas de la méthode DMA, le signal est filtré par un filtre passe bas dépendant de '$N$'. En effet, la réponse impulsionnelle du filtre est définie par $h_{DMA}(n) = \frac{1}{N}$ pour $n$ = 0, ..., $N$ - 1.

Étape 3 : La tendance globale obtenue est retranchée au profil. Une grandeur $F(N)$ est ensuite calculée comme la racine carrée de la puissance de la différence entre le signal intégré et sa tendance globale relative à $N$.

Étape 4 : Les étapes 2 et 3 sont répétées pour différentes valeurs de $N$. Puisque $F(N) \propto N^{\alpha}$ avec $\alpha = H + 1$ dû à l'intégration du signal, log($F(N)$) est représenté en fonction de log($N$). La valeur de la pente de la droite de régression des points obtenus est alors une estimation de $\alpha$ et donc de la régularité du signal. Une représentation d'une droite de régression obtenue à partir de trois tendances de profil est illustrée sur la partie droite de la figure 5.

Ces deux approches DFA et DMA qui ont été largement appliquées dans le domaine biomédical pour l'étude de pathologies dans la voix, des signaux EEG et ECG, ont montré de bonnes performances dans l'estimation du coefficient de Hurst. Leur utilisation reste plus limitée dans d'autres domaines comme dans un contexte industriel. En météorologie, un exemple est la demande de brevet WO2017/132225 A1 «Weather-based industry analysis system».

Il existe des variantes de ces approches dont le but est de leur apporter des correctifs. Pour la méthode DFA, on peut citer par exemple les DFA d'ordre supérieur à 1, tels que « l'Adaptative Fractal Analysis » (AFA) ou citer J. W. Kantelhardt, E. Koscielny-Bunde, H. H. A. Rego, S. Havlin et A. Bunde dans «Detecting long-range correlations with detrended fluctuation analysis,» Physica A: Statistical Mechanics and its Applications, n° %1295, pp. 441-454, 2001 qui propose une étude qui vise à ajouter un terme correctif pour les petites valeurs de *N*.

Pour la méthode DMA, on peut citer les variantes « Centered DMA », « Weigthed DMA d'ordre I » (WDMA-I), « Weigthed Centered DMA d'ordre I » (WCDMA-I).

Il ressort que dans les contextes d'application connus, les techniques de rehaussement de signal ne permettent pas de conserver la régularité du signal. Avantageusement, le procédé de la présente invention ne procède pas à un rehaussement du signal pour supprimer le bruit, et ensuite estimer la régularité du signal d'intérêt, mais il estime directement la régularité du signal d'intérêt à partir du signal bruité en prenant en compte l'influence du bruit additif de mesure, sans perdre l'information d'intérêt, ni altérer la régularité du signal. Le procédé de l'invention s'affranchit de l'étape de rehaussement des méthodes usuelles, de manière à compenser directement l'influence du bruit de mesure dans le calcul de la puissance de la différence entre le signal bruité intégré et sa tendance.

Ainsi, revenant à la figure 2, l'analyse du signal bruité selon la présente invention consiste à estimer (206) la régularité du signal d'intérêt avec une compensation directe de l'influence du bruit de mesure dans l'analyse.

Dans une étape suivante (208), le procédé permet de déterminer l'état du système selon le résultat de l'analyse de régularité du signal d'intérêt.

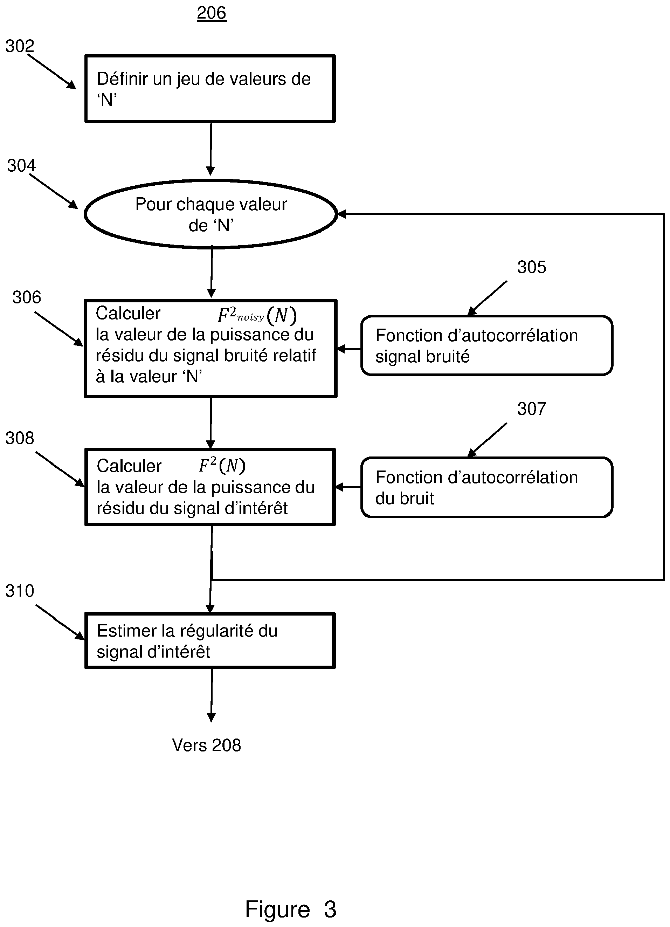

La figure 3 qui détaille l'étape (206), illustre un enchaînement d'étapes pour estimer la régularité d'un signal d'intérêt à partir d'un signal bruité avec compensation de l'influence du bruit, selon un mode de réalisation de l'invention basé sur la méthode DFA. Cependant, le procédé peut être transposé pour toute autre méthode d'estimation de régularité reposant sur un calcul de tendance du signal.

Le procédé permet dans une première étape (302) de définir un jeu de valeurs de *N*, correspondant à un découpage du signal bruité en segments de taille '*N*', puis va réitérer (304) plusieurs étapes (306 à 308) pour chaque valeur *de N*.

Dans l'approche classique du DFA, $F.(N)$ est défini selon l'équation suivante :

$$F.(N) = \sqrt{\sum_{l=1}^{L}\sum_{n=1}^{N}\left(yint\left((l-1)N+n\right)-x_l(n)\right)^2}$$

avec $x_l(n)$ le *n*-ieme échantillon de la *l*-ième tendance locale.

Il est également possible d'exprimer $F.(N)$ de manière matricielle selon l'équation suivante:

$$F.^2(N) = \sum_{k=1}^{LN}\Gamma_{N,\cdot}(k,k)y^2(k) + 2\sum_{r=1}^{LN-1}\sum_{k=1}^{LN-r}\Gamma_{N,\cdot}(k,k+r)y(k)y(k+r)$$

où $\Gamma_{N,\cdot}(k,k+r)$ désigne l'élément de la matrice $\Gamma_{N,\cdot}$ situé à la ligne *k* et à la colonne *k* + *r* et avec $F_{\cdot}^2(N)$ correspondant à la puissance de la différence entre le signal d'intérêt intégré et sa tendance relative à la méthode choisie (DFA, DMA, etc.) et $\Gamma_{N,\cdot}$ une matrice appropriée.

Dans l'étape (306), le procédé permet de calculer $F^2_{\cdot,noisy}(N)$, la valeur de la puissance de la différence entre le signal bruité intégré et sa tendance relative à la valeur 'N' en cours. Cela consiste à exprimer $F.(N)$ en fonction des échantillons du signal bruité : $z(k) = y(k) + b(k)$

Etant donné le développement mathématique ci-dessus, il vient que la valeur de la puissance de la différence entre le signal bruité intégré et sa tendance relative à la valeur 'N' en cours, peut s'exprimer comme suit :

$$F^2_{\cdot,noisy}(N) = \sum_{k=1}^{LN}\Gamma_{N,\cdot}(k,k)z^2(k) + 2\sum_{r=1}^{LN-1}\sum_{k=1}^{LN-r}\Gamma_{N,\cdot}(k,k+r)z(k)z(k+r)$$

Cette expression peut être approximée selon la fonction d'autocorrélation du signal bruité (305).

En effet, mathématiquement, il est possible d'écrire :

$$E[F_{\cdot,noisy}^2(N)] = \sum_{k=1}^{LN} \Gamma_{N,\cdot}(k,k)E[z^2(k)] + 2\sum_{r=1}^{LN-1}\sum_{k=1}^{LN-r} \Gamma_{N,\cdot}(k,k+r)E[z(k)z(k+r)]$$

avec *E*[] l'opérateur espérance mathématique. De ce fait, dans le cas d'un processus stationnaire au sens large, cette équation devient :

$$E[F_{\cdot,noisy}^2(N)] = \sum_{k=1}^{LN} \Gamma_{N,\cdot}(k,k)R_{z,z}(0) + 2\sum_{r=1}^{LN-1}\sum_{k=1}^{LN-r} \Gamma_{N,\cdot}(k,k+r)\,R_{z,z}(r)$$

Finalement, pour un processus stationnaire et ergodique, il est possible d'approximer la valeur de $F_{\cdot,noisy}^2(N)$ par $E\left[F_{\cdot,noisy}^2(N)\right]$. L'homme de l'art comprend que cela revient à approximer le produit $z(k)z(k + r)$ par l'autocorrélation $R_{z,z}(r)$ prise pour un retard $r$ avec $r = 0, ..., LN - 1$.

Dans ce cas, il vient :

$$F_{\cdot,noisy}^2(N) \approx \sum_{k=1}^{LN} \Gamma_{N,\cdot}(k,k)\hat{R}_{z,z}(0) + 2\sum_{r=1}^{LN-1}\sum_{k=1}^{LN-r} \Gamma_{N,\cdot}(k,k+r)\,\hat{R}_{z,z}(r) = \hat{F}_{\cdot,noisy}^2(N)$$

avec $\widehat{R_{z,z}}$ l'estimée de la fonction d'autocorrélation du signal bruité.
Cette expression peut être réécrite selon l'équation suivante :

$$\hat{F}_{\cdot,noisy}^2(N) = Tr(\Gamma_{N,\cdot})\hat{R}_{z,z}(0) + 2\sum_{r=1}^{LN-1} Tr(\Gamma_{N,\cdot},r)\hat{R}_{z,z}(r)$$

Où $Tr(\Gamma_{N,\cdot},r)$ désigne la trace de la rème diagonale de $\Gamma_{N,\cdot}$.

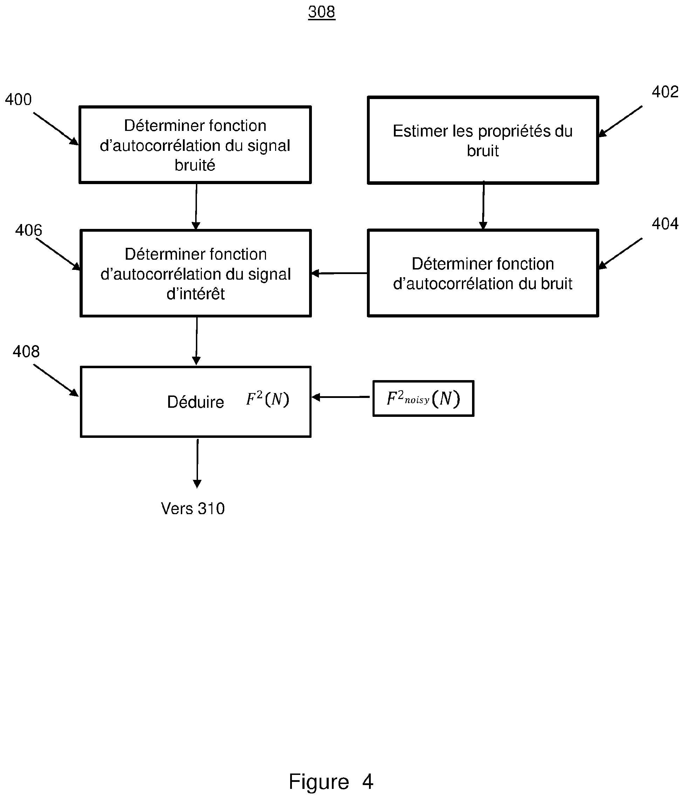

Dans l'étape suivante (308), le procédé permet de calculer la valeur de la puissance de la différence entre le signal d'intérêt intégré $\hat{F}_{\cdot}^2(N)$ en compensant la contribution du bruit dans le calcul, selon la fonction d'autocorrélation du signal d'intérêt (307), comme détaillé par la description de la figure 4.

Les étapes 306 à 308 sont répétées pour chaque valeur de *N*, et quand toutes les valeurs de *N* valeurs sont traitées, le procédé permet dans une étape suivante (310) d'estimer la régularité du signal d'intérêt à partir du signal bruité, puis de continuer avec l'étape de détermination de l'état du système (208).

La figure 4 détaille l'étape (308) permettant de déduire la valeur de la puissance de la différence entre le signal d'intérêt intégré et la tendance $\hat{F}_{\cdot}^2(N)$ avec compensation de l'influence du bruit. Dans une étape (402), le procédé permet de d'estimer les propriétés du bruit, puis de déterminer (404) l'expression de la fonction d'autocorrélation du bruit, notée $R_{b,b}(r)$.

L'homme du métier comprend que les propriétés du bruit à estimer peuvent porter par exemple sur les propriétés statistiques et/ou la nature colorée ou blanche du bruit et/ou la modélisation paramétrique qui peut en être faite, etc.

A partir de la fonction d'autocorrélation du bruit (404) et de la fonction d'autocorrélation du signal bruité (400), le procédé permet de déterminer (406) la fonction d'autocorrélation du signal d'intérêt.

Dans le cas où le bruit et le signal sont non corrélés et stationnaires, la fonction d'autocorrélation du signal bruité *z* s'exprime comme la somme de la fonction d'autocorrélation du signal d'intérêt *y* et celle du bruit additif *b* selon l'équation suivante :

$$\hat{R}_{y,y}(r) = \hat{R}_{z,z}(r) - \hat{R}_{b,b}(r) \text{ avec } r = 0, \ldots, LN-1.$$

Puis dans une étape suivante (408), le procédé permet de compenser mathématiquement l'influence du bruit dans le calcul de la valeur de la puissance de la différence entre le signal d'intérêt intégré et la tendance $\hat{F}^2(N)$.

Le procédé continue à l'étape (310) pour estimer la régularité du signal d'intérêt.

Un exemple est donné avec un bruit blanc : soit *b* un bruit blanc gaussien de moyenne nulle et de variance $\sigma_b^2$. Dans ce cas, le signal bruité *z* est défini comme suit :

$$z(n) = y(n) + b(n).$$

La fonction d'autocorrélation de *b* étant égale à $R_{b,b}(r) = \sigma_b^2 \delta(r)$ avec la fonction δ définie par :

$$\delta(r) = \begin{cases} 1 \text{ si } r = 0 \\ 0 \quad \text{sinon} \end{cases},$$

il vient :

$$R_{y,y}(r) = R_{z,z}(r) - \sigma_b^2 \delta(r).$$

Il est alors possible d'exprimer $F_{\cdot,noisy}^2(N)$, la puissance de la différence entre le signal bruité intégré et la tendance estimée associée à *N,* en fonction de la puissance de la différence entre le signal d'intérêt intégré et la tendance estimée sur ce même signal d'intérêt intégré associée à *N*, selon l'équation :

$$F_{\cdot}^2(N) \approx F_{\cdot,noisy}^2(N) - Tr(\Gamma_{N},.)\sigma_u^2$$

avec $Tr(\Gamma_{N},.)$ la trace de la matrice $\Gamma_{N},.$, *i.e.* la somme de ses éléments diagonaux.

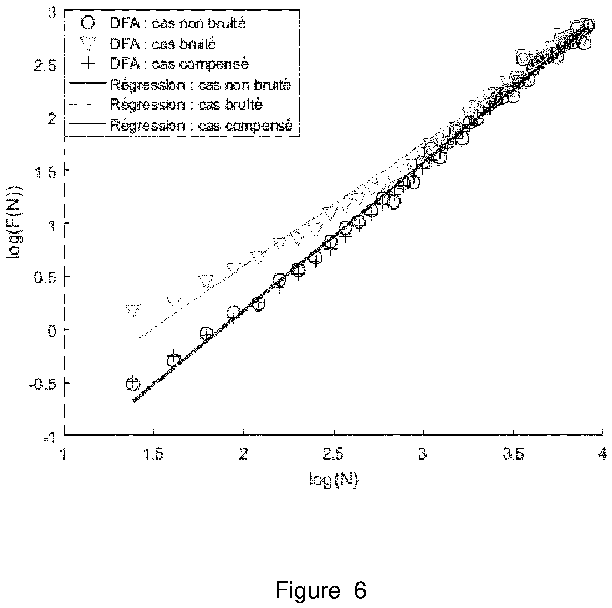

La figure 6 illustre sur un graphe log(F(N)) en fonction de log(N), l'évolution de différentes valeurs de $\hat{F}_{\cdot}^2(N)$ en fonction de *N* par la méthode DFA. Le signal a une régularité estimée à $\alpha$ = 1.60. En bruitant ce signal avec un bruit blanc de même puissance que le signal, on peut constater que le bruit modifie de manière significative la valeur de $F_{\cdot,noisy}^2(N)$ pour les petites valeurs de *N.* La valeur de $\alpha$ estimée dans le cas bruité en est donc altérée et est estimée à $\alpha_{noisy}$ = 1.35. En utilisant la méthode de compensation du bruit de la présente invention, il est possible de supprimer cette contribution dans le calcul de $\hat{F}_{\cdot}^2(N)$, et la valeur de $\alpha$ estimée est améliorée. Dans le cas décrit en exemple, elle vaut : $\alpha_{compensé}$ = 1.61.

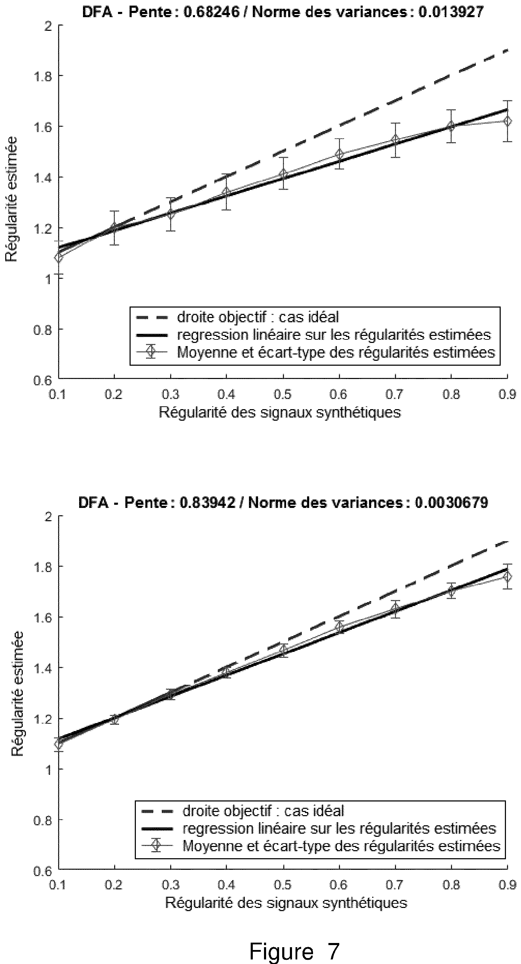

La figure 7 illustre des comparaisons de résultats obtenus pour le graphe du haut par une approche classique de rehaussement de signal avant l'estimation de la régularité et pour le graphe du bas par la méthode de compensation du bruit de l'invention. L'exemple est montré pour 50 signaux synthétiques dont la régularité est connue, et allant de 0.1 à 0.9. Ces signaux sont ensuite bruités avec un bruit blanc dont la puissance est égale à celle du signal.

La courbe du haut montre les résultats quand les signaux sont d'abord rehaussés puis que la régularité est estimée sur le signal rehaussé ou débruité, par une approche classique. Il peut alors être constaté que pour les hautes valeurs de $\alpha$, le fait de rehausser un signal altère la valeur de régularité estimée. Ce phénomène s'accentue lorsque la régularité a priori du signal de base augmente.

La courbe du bas montre les résultats avec une compensation directe du bruit lors de l'estimation de la régularité selon la méthode de l'invention. Il peut alors être constaté que le phénomène d'altération de la valeur de régularité estimée est atténué, et que le procédé de l'invention permet ainsi de mieux estimer en moyenne la régularité d'un signal bruité.

De plus, la variance d'estimation avec la méthode de compensation de bruit de l'invention est également plus faible que celle obtenue avec l'approche de rehaussement classique. Avantageusement, il peut alors être accordé plus de confiance à la valeur de régularité $\alpha$ estimée sur un signal réel par le procédé de l'invention que celle par les approches connues. Cette méthode permet ainsi une estimation de la régularité d'un signal plus robuste et plus précise.

## Revendications

1. Procédé (200) mis en œuvre par ordinateur pour déterminer l'état d'un système, le procédé comprenant des étapes consistant à :

   - collecter (202) des données relatives à un système, lesdites données étant des données bruitées comprenant des données utiles et du bruit ;
   - générer (204) un signal à analyser à partir des données collectées, ledit signal étant un signal bruité comprenant un signal d'intérêt et du bruit ;
   - effectuer (206) une analyse de régularité du signal d'intérêt à partir du signal bruité en compensant l'influence du bruit dans le calcul de la puissance de la différence entre le signal bruité intégré et sa tendance; et
   - déterminer (208) l'état dudit système en fonction du résultat de l'analyse de régularité du signal d'intérêt estimée à partir du signal bruité.

2. Le procédé selon la revendication 1 dans lequel l'étape (206) d'analyse de régularité du signal bruité comprend les étapes de :

   - définir (302) un jeu de valeurs de 'N' :

     - pour chaque valeur de 'N' (304):

       - calculer (306) la valeur de la puissance de la différence entre le signal bruité intégré et sa tendance estimée relative à la valeur 'N' à partir de la fonction d'autocorrélation du signal bruité (305) ;
       - calculer (308) la valeur de la puissance de la différence entre le signal d'intérêt intégré et sa tendance à partir de la différence entre le signal bruité intégré et sa tendance estimée relative à la valeur 'N' et de la fonction d'autocorrélation du bruit (307); et

     - estimer (310) la régularité du signal bruité.

3. Le procédé selon la revendication 1 ou 2 comprenant après l'étape (204) de générer un signal à analyser, une étape (400) de calculer une fonction d'autocorrélation du signal bruité.

4. Le procédé selon la revendication 3 dans lequel l'étape (308) de calculer la valeur de la puissance de la différence entre le signal d'intérêt intégré et sa tendance comprend les étapes de :

   - estimer (402) les propriétés du bruit ;
   - calculer (404) la fonction d'autocorrélation du bruit ;
   - calculer (406) la fonction d'autocorrélation du signal d'intérêt à partir de la fonction d'autocorrélation du bruit et de la fonction d'autocorrélation du signal bruité ; et
   - compenser mathématiquement par la fonction d'autocorrélation du bruit l'influence du bruit dans le calcul (408) de la valeur de la puissance de la différence entre le signal d'intérêt intégré et sa tendance.

5. Le procédé selon la revendication 4, dans lequel l'étape d'estimation des propriétés du bruit consiste à estimer les propriétés statistiques et/ou la nature colorée ou blanche du bruit et/ou les paramètres d'une modélisation paramétrique qui serait faite dudit bruit.

6. Le procédé selon l'une quelconque des revendications 2 à 5 dans lequel l'étape (302) de définir un jeu de valeurs de 'N' consiste à découper le signal bruité intégré en segments de taille 'N' et l'étape (310) d'estimer la régularité du signal d'intérêt peut consister à inclure l'estimation de la tendance dudit signal intégré pour le jeu de valeurs de 'N'.

7. Le procédé selon l'une quelconque des revendications 2 à 5 dans lequel l'étape (302) de définir un jeu de valeurs de 'N' consiste à appliquer un filtre passe-bas d'ordre 'N' au signal bruité intégré et l'étape (310) d'estimer la régularité du signal d'intérêt peut consister à inclure l'estimation de la tendance dudit signal intégré.

8. Le procédé selon l'une quelconque des revendications précédentes dans lequel l'étape (202) de collecter des données, consiste à collecter des données depuis des capteurs couplés au système.

9. Le procédé selon la revendication 8 dans lequel les capteurs sont configurés pour relever des données relatives à la réponse électrique du cerveau (EEG) « ElectroEncephaloGramme » et/ou à la réponse électrique du cœur (ECG) « ElectroCardioGramme » et/ou à la réponse électrodermale et/ou à la pupillométrie.

**10.** Le procédé selon la revendication 8 ou 9 dans lequel les capteurs comprennent au moins un capteur inertiel qui comprend des accéléromètres et des gyromètres.

**11.** Le procédé selon l'une quelconque des revendications 1 à 10 comprenant de plus une étape consistant à afficher l'état dudit système en fonction du résultat de l'analyse de régularité.

**12.** Produit programme d'ordinateur, ledit programme d'ordinateur comprenant des instructions de code non transitoire permettant d'effectuer les étapes du procédé selon l'une quelconque des revendications 1 à 11, lorsque ledit programme est exécuté sur un ordinateur.

**13.** Dispositif (100) pour déterminer l'état d'un système, le dispositif comprenant :

- des moyens (102) pour collecter des données relatives à un système (101), lesdites données étant des données bruitées comprenant des données d'intérêt et du bruit ;
- des moyens (104) pour générer un signal à analyser à partir des données collectées, ledit signal étant un signal bruité comprenant un signal d'intérêt et du bruit ;
- des moyens (106) pour effectuer une analyse de régularité du signal d'intérêt en compensant l'influence du bruit dans le calcul de la puissance de la différence entre le signal bruité intégré et sa tendance ; et
- des moyens (108) pour déterminer l'état dudit système en fonction du résultat de l'analyse de régularité.

**14.** Le dispositif selon la revendication 13 comprenant de plus une interface utilisateur (110) configurée pour fournir l'état du système.

**15.** Le dispositif selon la revendication 13 ou 14 dans lequel le système est une personne ou un équipement.

101
Système
100
102
Acquisition données bruitées
104
Génération d'un signal à analyser
106
Estimation de la régularité du signal d'intérêt avec compensation du bruit
108
Détermination de l'état du système
110
Interface utilisateur I/O

Figure 1

200
202
Collecter des données bruitées relatives à un système
204
Générer un signal à analyser composé d'un signal d'intérêt et de bruit
206
Analyser la régularité du signal d'intérêt avec compensation de l'influence du bruit
208
Déterminer l'état du système

Figure 2

206
302
Définir un jeu de valeurs de 'N'
304
Pour chaque valeur de 'N'
305
306
Calculer $F^2{}_{noisy}(N)$ la valeur de la puissance du résidu du signal bruité relatif à la valeur 'N'
Fonction d'autocorrélation signal bruité
307
308
Calculer $F^2(N)$ la valeur de la puissance du résidu du signal d'intérêt
Fonction d'autocorrélation du bruit
310
Estimer la régularité du signal d'intérêt
Vers 208

Figure 3

308
400
Déterminer fonction d'autocorrélation du signal bruité
Estimer les propriétés du bruit
402
406
Déterminer fonction d'autocorrélation du signal d'intérêt
Déterminer fonction d'autocorrélation du bruit
404
408
Déduire $F^2(N)$
$F^2{}_{noisy}(N)$
Vers 310

Figure 4

$N = 40$
$N = 20$
$N = 10$
Pente = $\alpha$
$log(F(N))$
$log(N)$
$F(N) \propto N^{\alpha}$
$\log(F(N)) \propto \alpha * \log(N)$


Figure 5

DFA : cas non bruité
DFA : cas bruité
DFA : cas compensé
Régression : cas non bruité
Régression : cas bruité
Régression : cas compensé
log(F(N))
log(N)
3
2.5
2
1.5
1
0.5
0
-0.5
-1
1
1.5
2
2.5
3
3.5
4

Figure 6

DFA - Pente : 0.68246 / Norme des variances : 0.013927
Régularité estimée
Régularité des signaux synthétiques
droite objectif : cas idéal
regression linéaire sur les régularités estimées
Moyenne et écart-type des régularités estimées
2
1.8
1.6
1.4
1.2
1
0.8
0.6
0.1
0.2
0.3
0.4
0.5
0.6
0.7
0.8
0.9

DFA - Pente : 0.83942 / Norme des variances : 0.0030679
Régularité estimée
Régularité des signaux synthétiques
droite objectif : cas idéal
regression linéaire sur les régularités estimées
Moyenne et écart-type des régularités estimées
2
1.8
1.6
1.4
1.2
1
0.8
0.6
0.1
0.2
0.3
0.4
0.5
0.6
0.7
0.8
0.9

Figure 7

## RAPPORT DE RECHERCHE EUROPEENNE

Numéro de la demande
EP 20 17 1261

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 5 768 392 A (GRAUPE DANIEL [US]) 16 juin 1998 (1998-06-16)<br>* abrégé *<br>* colonne 3, ligne 22 - ligne 50 *<br>* colonne 4, ligne 54 - ligne 67 *<br>* colonne 5, ligne 5 - ligne 10 *<br>* colonne 5, ligne 32 - ligne 45 *<br>* colonne 7, ligne 24 - colonne 8, ligne 13 *<br>* colonne 7, ligne 10 *<br>* colonne 8, ligne 18 *<br>* colonne 8, ligne 45 - ligne 49 *<br>* colonne 10, ligne 29 - ligne 64 *<br>* colonne 10, ligne 41 - ligne 42 *<br>* colonne 10 - ligne 45 *<br>* revendication 11 *<br>* figure 9 *<br>----- | 1-15 | INV.<br>A61B5/04<br>A61B5/0402<br>A61B5/048<br>A61B5/16<br>A61B5/00 |
| A | TATJANA STADNITSKI: "Measuring Fractality",<br>FRONTIERS IN PHYSIOLOGY,<br>vol. 3, 1 janvier 2012 (2012-01-01),<br>XP055668770,<br>DOI: 10.3389/fphys.2012.00127<br>* abrégé *<br>* figure 2 *<br>-----<br>-/-- | 1-15 | |
| | | | **DOMAINES TECHNIQUES RECHERCHES (IPC)** |
| | | | A61B<br>G10K |

Le présent rapport a été établi pour toutes les revendications

| Lieu de la recherche | Date d'achèvement de la recherche | Examinateur |
|---|---|---|
| La Haye | 16 juillet 2020 | Delval, Christophe |

1

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
& : membre de la même famille, document correspondant

EPO FORM 1503 03.82 (P04C02)

RAPPORT DE RECHERCHE EUROPEENNE

Numéro de la demande
EP 20 17 1261

**DOCUMENTS CONSIDERES COMME PERTINENTS**

| Catégorie | Citation du document avec indication, en cas de besoin, des parties pertinentes | Revendication concernée | CLASSEMENT DE LA DEMANDE (IPC) |
|---|---|---|---|
| A | SHING-HONG LIU ET AL: "ECG Noise Cancellation Based on Grey Spectral Noise Estimation", SENSORS, vol. 19, no. 4, 15 février 2019 (2019-02-15), page 798, XP055668807, DOI: 10.3390/s19040798 * abrégé * * sect."Discussion"; page 12 - page 13 * * le document en entier * ----- | 1-15 | |
| A | Aarti Singh: "Adaptative Noise Cancellation", , 1 janvier 2011 (2011-01-01), XP055668810, Extrait de l'Internet: URL:http://www.cs.cmu.edu/~aarti/pubs/ANC.pdf [extrait le 2020-02-14] * abrégé * * sect. II * * le document en entier * ----- | 1-15 | DOMAINES TECHNIQUES RECHERCHES (IPC) |
| A | BANERJEE SWATI ET AL: "A cross wavelet transform based approach for ECG feature extraction and classification without denoising", PROCEEDINGS OF THE 2014 INTERNATIONAL CONFERENCE ON CONTROL, INSTRUMENTATION, ENERGY AND COMMUNICATION (CIEC), IEEE, 31 janvier 2014 (2014-01-31), pages 162-165, XP032684279, DOI: 10.1109/CIEC.2014.6959070 * abrégé * * le document en entier * ----- -/-- | 1-15 | |

Le présent rapport a été établi pour toutes les revendications

1

| Lieu de la recherche | Date d'achèvement de la recherche | Examinateur |
|---|---|---|
| La Haye | 16 juillet 2020 | Delval, Christophe |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

EPO FORM 1503 03.82 (P04C02)

# RAPPORT DE RECHERCHE EUROPEENNE

Numéro de la demande
EP 20 17 1261

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | CN 107 132 033 A (STATE GRID CORP CHINA; STATE GRID HUNAN ELECTRIC POWER CO ET AL.) 5 septembre 2017 (2017-09-05) * abrégé * * revendications 6,7 * * le document en entier * ----- | 1-15 | |

DOMAINES TECHNIQUES RECHERCHES (IPC)

Le présent rapport a été établi pour toutes les revendications

| Lieu de la recherche | Date d'achèvement de la recherche | Examinateur |
|---|---|---|
| La Haye | 16 juillet 2020 | Delval, Christophe |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.......................................................................
& : membre de la même famille, document correspondant

1

EPO FORM 1503 03.82 (P04C02)

page 3 de 3

## ANNEXE AU RAPPORT DE RECHERCHE EUROPEENNE RELATIF A LA DEMANDE DE BREVET EUROPEEN NO.

EP 20 17 1261

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

16-07-2020

| Document brevet cité au rapport de recherche | Date de publication | Membre(s) de la famille de brevet(s) | Date de publication |
|---|---|---|---|
| US 5768392 A | 16-06-1998 | AUCUN | |
| CN 107132033 A | 05-09-2017 | AUCUN | |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2017132225 A1


### Littérature non-brevet citée dans la description


- **C. K. PENG ; S. V. BULDYREV ; S. HAVLIN ; M. SIMONS ; H. E. STANLEY ; A. L. GOLDBERGER.** Mosaic organization of DNA nucleotides. *Physical Review,* 1994, 1685-1689
- **D. OSBORNE.** Moving average detrending and the analysis of business cycles. *Oxford Bull. Econom. Statist,* 1995, 547-558
- **J. W. KANTELHARDT ; E. KOSCIELNY-BUNDE ; H. H. A. REGO ; S. HAVLIN ; A. BUNDE.** Detecting long-range correlations with detrended fluctuation analysis. *Physica A: Statistical Mechanics and its Applications,* 2001, 441-454